# EUROPEAN PATENT APPLICATION

(11) **EP 4 733 327 A1**
(43) Date of publication of application: **29.04.2026**
(21) Application number: 24830695.3
(22) Date of filing: 24.06.2024
(51) Int. Cl.: C08B 37/08, A61K 8/73, A61Q 19/00

(54) **GRAFTED COMPOUND OF HYALURONIC ACID OR SALT THEREOF AND GLUTAMIC ACID OR SALT THEREOF, AND PREPARATION METHOD THEREFOR AND USE THEREOF**

(30) Priority: 25.06.2023 CN 202310752350
(71) Applicant: Bloomage Biotechnology Corporation Limited, Jinan, Shandong 250101 (CN)
(72) Inventor: HAN, Dong, Jinan, Shandong 250101 (CN); WEI, Jian, Jinan, Shandong 250101 (CN); LIU, Zhe, Jinan, Shandong 250101 (CN); JIANG, Fangru, Jinan, Shandong 250101 (CN)
(74) Representative: Abel & Imray LLP
(86) International application number: PCT/CN2024/100955
(87) International publication number: WO 2025/002051

(57) **Abstract**

The present application provides a grafted compound of hyaluronic acid or a salt thereof and glutamic acid or a salt thereof, and a preparation method therefor. It is found that the graft has a synergistic effect in inhibiting IL-6 expression and can prevent or alleviate skin discomfort caused by inflammation.

## Description

### TECHNICAL FIELD

The present application relates to the field of cosmetics, and specifically to a graft compound of hyaluronic acid or a salt thereof and glutamic acid or a salt thereof, the preparation method and use thereof.

### BACKGROUND ART

In the skincare products formulations, hyaluronic acid or a salt thereof is often mixed with other active ingredients for use. However, in the case of mixing, there may be instances where the efficacy of the active ingredients cannot be fully exerted.

### SUMMARY OF THE INVENTION

The inventors of the present application have discovered that, the graft compound of hyaluronic acid or a salt thereof and glutamic acid or a salt thereof having a certain structure can effectively suppress the expression of IL-6, thereby accomplishing the present application.

The present application relates to the following solutions:
1. A graft compound of hyaluronic acid or a salt thereof and glutamic acid or a salt thereof, wherein at least a portion of the carboxyl groups of the hyaluronic acid or a salt thereof are linked to the amino groups of the glutamic acid or a salt thereof via amide bonds.
2. The graft compound according to item 1, having a general formula as shown in the following Formula (I): 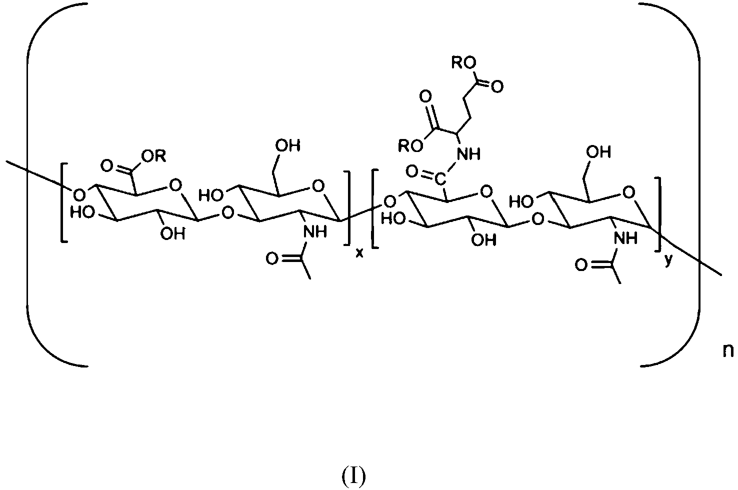 wherein in Formula (I), R is a metal ion or H, 0≤x< 1, 0<y≤ 1, x+y=1, n is a positive integer of 1 to 2000.
3. The graft compound according to item 1, wherein the grafting ratio of the graft compound is 20% to 100%.
4. The graft compound according to item 1, wherein the molecular weight of the graft compound is 3kDa to 500kDa.
5. The graft compound according to item 1, wherein in the ¹H NMR spectrum obtained by nuclear magnetic resonance detection of the graft compound, the graft compound has a characteristic peak at chemical shift δ of 4.00-4.15 ppm;
   preferably, in the ¹H NMR spectrum obtained by nuclear magnetic resonance detection of the graft compound, the graft compound has characteristic peaks at chemical shift δ of 2.10-2.25ppm and 1.71-2.10ppm.
6. A method for preparing the graft compound of hyaluronic acid or a salt thereof and glutamic acid or a salt thereof according to any one of items 1 to 5, wherein,
   the quaternary ammonium salt of hyaluronic acid or a salt thereof is prepared by: mixing hyaluronic acid or a salt thereof with a quaternary ammonium base to react to obtain the quaternary ammonium salt of hyaluronic acid or a salt thereof;
   the graft compound is prepared by: dissolving the quaternary ammonium salt of hyaluronic acid or a salt thereof, then adding glutamic acid or a salt thereof, activator and catalyst for reaction, hydrolyzing the resulting mixture under alkaline condition, and then adjusting pH, thereby obtaining the graft compound of hyaluronic acid or a salt thereof and glutamic acid or a salt thereof.
7. A cosmetic composition, wherein the cosmetic composition comprises the graft compound according to any one of items 1 to 5 or the graft compound prepared by the method of item 6.
8. Use of the graft compound according to any one of items 1 to 5 or the graft compound prepared by the method of item 6 in cosmetics.
9. Use of the graft compound according to any one of items 1 to 5 or the graft compound prepared by the method of item 6 or the cosmetic composition according to item 7 in preventing and/or alleviating skin discomfort caused by inflammation.
10. Use of the graft compound according to any one of items 1 to 5 or the graft compound prepared by the method of item 6 or the cosmetic composition according to item 7 in anti-inflammation.

Compared to the prior art, the present application has the following beneficial effects:
The graft compound of hyaluronic acid or a salt thereof and glutamic acid or a salt thereof provided herein can effectively exert anti-inflammatory and skincare benefits, and expand the application range of the graft compounds.
Furthermore, the present application has obtained the preferred structure of this new raw material when it is used to inhibit the expression of IL-6.

### BRIEF DESCRIPTION OF THE FIGURES

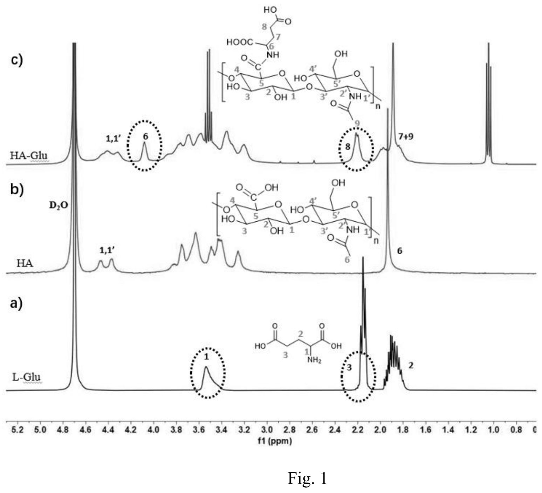

FIG. 1 shows the ¹H NMR spectrum of Example 6.

### DETAIL DESCRIPTION OF THE INVENTION

The following examples are used in combination to further describe the present application. It should be understood that the examples are only used to further illustrate and explain the present application and are not intended to limit the present application.

Unless otherwise defined, the technical and scientific terms used in the description of the present application shall have the meanings commonly understood by those skilled in the art. Although similar or identical methods and materials may be applied in experiments or practical applications, the materials and methods are described herein below. In case of conflict, the definitions contained herein shall prevail. Furthermore, the materials, methods, and examples are provided for illustrative purposes only and are not intended to be limiting. The following contents further illustrate the present application in combination with the specific examples, but they are not intended to limit the scope of the present application.

Provided herein is a graft compound of hyaluronic acid or a salt thereof and glutamic acid or a salt thereof, wherein at least a portion of the carboxyl groups of hyaluronic acid or a salt thereof are linked to the amino groups of glutamic acid or a salt thereof via amide bonds.

The graft compound of hyaluronic acid or a salt thereof and glutamic acid or a salt thereof described herein can enable hyaluronic acid or a salt thereof and glutamic acid or a salt thereof to act simultaneously on skin cells, thus achieving superior efficacy compared to their physical mixtures.

The degree of substitution refers to the molar ratio of glutamic acid or a salt thereof which are linked to the disaccharide structure of hyaluronic acid or a salt thereof via amide bond, to the disaccharide structure of hyaluronic acid or a salt thereof.

Wherein hyaluronates encompass unmodified hyaluronates or modified hyaluronates (e.g., acetylated hyaluronates). Examples may be sodium salts, zinc salts, calcium salts, magnesium salts and the like of hyaluronic acid, or any combination of these salts.

In some embodiments described herein, the hyaluronic acid or a salt thereof has a molecular weight of 2.5kDa to 500kDa. For example, the hyaluronic acid or a salt thereof can have a molecular weight of 2.5 kDa, 2.6 kDa, 2.7 kDa, 2.8 kDa, 2.9 kDa, 3kDa, 5kDa, 10kDa, 15kDa, 20kDa, 25kDa, 30kDa, 35kDa, 40kDa, 45kDa, 50kDa, 55kDa, 60kDa, 65kDa, 70kDa, 75kDa, 80kDa, 85kDa, 90kDa, 95kDa, 100kDa, 110kDa, 120kDa, 130kDa, 140kDa, 150kDa, 160kDa, 170kDa, 180kDa, 190kDa, 200kDa, 210kDa, 220kDa, 230kDa, 240kDa, 250kDa, 260kDa, 270kDa, 280kDa, 290kDa, 300kDa, 310kDa, 320kDa, 330kDa, 340kDa, 350kDa, 360kDa, 370kDa, 380kDa, 390kDa, 400kDa, 410kDa, 420kDa, 430kDa, 440kDa, 450kDa, 460kDa, 470kDa, 480kDa, 490kDa, 500kDa, or any range therebetween. Glutamates encompass unmodified glutamates or modified glutamates. Examples may be hydrochlorides, acetates, sulfonates and the like of glutamic acid, or any combination of these salts.

In some embodiments, the graft compound has a general formula as shown in the following Formula (I): 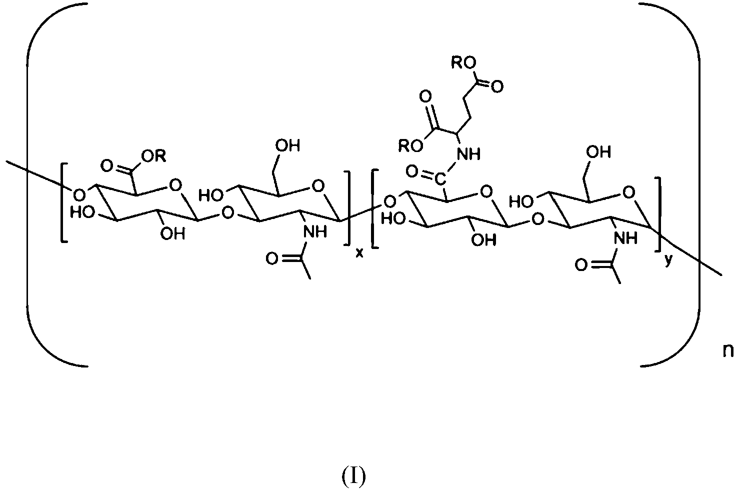 wherein in Formula (I), R is a metal ion or H, 0≤x< 1, 0<y≤ 1, x+y=1, n is a positive integer of 1 to 2000.

For example, x can be 0, 0.05, 0.1, 0.15, 0.2, 0.25, 0.3, 0.35, 0.4, 0.45, 0.5, 0.55, 0.6, 0.65, 0.7, 0.75, 0.8, 0.85, 0.9, 0.95, 0.99, or any numerical value therebetween.

y can be 0.01, 0.05, 0.1, 0.15, 0.2, 0.25, 0.3, 0.35, 0.4, 0.45, 0.5, 0.55, 0.6, 0.65, 0.7, 0.75, 0.8, 0.85, 0.9, 0.95, 1, or any numerical value therebetween.

n can be 1, 2, 3, 4, 5, 6, 7, 8, 9, 10, 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, 1000, 1100, 1200, 1300, 1400, 1500, 1600, 1700, 1800, 1900, 2000, or any positive integer therebetween.

In some embodiments described herein, the grafting ratio can be expressed as y/x+y.

In some embodiments described herein, the graft compound has a general formula as shown in the following Formula (II): 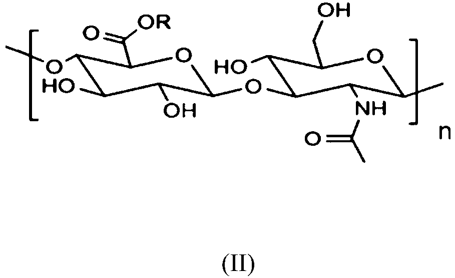 wherein in Formula (II), R is a metal ion or H, n is a positive integer of 1 to 2000, at least a portion of ORs are substituted by glutamic acid, and the substituent of glutamic acid is shown as in Formula (III): 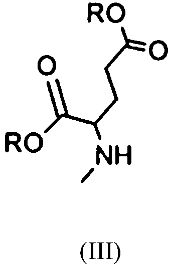 wherein in Formula (III), R is a metal ion or H.

In some embodiments described herein, the grafting ratio is measured by nuclear magnetic resonance spectroscopy ¹H NMR; wherein, the grafting ratio is calculated by the peak area integral of the methine group (-CH- proton) of glutamic acid or a salt thereof in the graft compound at the position shifted to 4.05-4.12 ppm in ¹H NMR spectrum obtained by nuclear magnetic resonance detection, or calculated by proton peak area integral of two methylene groups (-CH₂-) of glutamic acid or a salt thereof and methyl group (-CH₃-) of hyaluronic acid or a salt thereof in the graft compound in ¹H NMR spectrum. In some embodiments described herein, the grafting ratio of the graft compound is 20% to 100%, e.g., the grafting ratio of the graft compound can be 20%, 25%, 30%, 35%, 40%, 45%, 50%, 55%, 60%, 65%, 70%, 75%, 80%, 85%, 90%, 95%, 100%, or any range therebetween.

In some embodiments described herein, the molecular weight of the graft compound is 3kDa to 500kDa; for example, the molecular weight of the graft compound can be 3kDa, 5kDa, 10kDa, 15kDa, 20kDa, 25kDa, 30kDa, 35kDa, 40kDa, 45kDa, 50kDa, 55kDa, 60kDa, 65kDa, 70kDa, 75kDa, 80kDa, 85kDa, 90kDa, 95kDa, 100kDa, 110kDa, 120kDa, 130kDa, 140kDa, 150kDa, 160kDa, 170kDa, 180kDa, 190kDa, 200kDa, 210kDa, 220kDa, 230kDa, 240kDa, 250kDa, 260kDa, 270kDa, 280kDa, 290kDa, 300kDa, 310kDa, 320kDa, 330kDa, 340kDa, 350kDa, 360kDa, 370kDa, 380kDa, 390kDa, 400kDa, 410kDa, 420kDa, 430kDa, 440kDa, 450kDa, 460kDa, 470kDa, 480kDa, 490kDa, 500kDa, or any range therebetween.

In some embodiments described herein, in the ¹H NMR spectrum obtained by nuclear magnetic resonance detection of the graft compound, the graft compound has a characteristic peak at chemical shift δ of 4.00-4.15 ppm, e.g., the graft compound has a characteristic peak at chemical shift δ of 4.00ppm, 4.01ppm, 4.02ppm, 4.03ppm, 4.04ppm, 4.05ppm, 4.06ppm, 4.07ppm, 4.08ppm, 4.09ppm, 4.10ppm, 4.11ppm, 4.12ppm, 4.13ppm, 4.14ppm, 4.15ppm, or any range therebetween.

In some embodiments described herein, in the ¹H NMR spectrum obtained by nuclear magnetic resonance detection of the graft compound, the graft compound has a characteristic peak at chemical shift δ of 2.10-2.25 ppm, e.g., the graft compound has a characteristic peak at chemical shift δ of 2.10ppm, 2.11ppm, 2.12ppm, 2.13ppm, 2.14ppm, 2.15ppm, 2.16ppm, 2.17ppm, 2.18ppm, 2.19ppm, 2.20ppm, 2.21ppm, 2.22ppm, 2.23ppm, 2.24ppm, 2.25ppm, or any range therebetween.

In some embodiments described herein, in the ¹H NMR spectrum obtained by nuclear magnetic resonance detection of the graft compound, the graft compound has a characteristic peak at chemical shift δ of 1.71-2.10 ppm, e.g., the graft compound has a characteristic peak at chemical shift δ of 1.71ppm, 1.72ppm, 1.73ppm, 1.74ppm, 1.75ppm, 1.76ppm, 1.77ppm, 1.78ppm, 1.79ppm, 1.80ppm, 1.81ppm, 1.82ppm, 1.83ppm, 1.84ppm, 1.85ppm, 1.86ppm, 1.87ppm, 1.88ppm, 1.89ppm, 1.90ppm, 1.91ppm, 1.92ppm, 1.93ppm, 1.94ppm, 1.95ppm, 1.96ppm, 1.97ppm, 1.98ppm, 1.99ppm, 2.00ppm, 2.01ppm, 2.02ppm, 2.03ppm, 2.04ppm, 2.05ppm, 2.06ppm, 2.07ppm, 2.08ppm, 2.09ppm, 2.10ppm, or any range therebetween.

In some embodiments described herein, in the ¹H NMR spectrum obtained by nuclear magnetic resonance detection of the graft compound, the graft compound has a characteristic peak at chemical shift δ of 4.30-4.40 ppm, e.g., the graft compound has a characteristic peak at chemical shift δ of 4.30ppm, 4.31ppm, 4.32ppm, 4.33ppm, 4.34ppm, 4.35ppm, 4.36ppm, 4.37ppm, 4.38ppm, 4.39ppm, 4.40ppm, or any range therebetween.

In some embodiments described herein, in the ¹H NMR spectrum obtained by nuclear magnetic resonance detection of the graft compound, the graft compound has a characteristic peak at chemical shift δ of 4.30-4.48 ppm, e.g., the graft compound has a characteristic peak at chemical shift δ of 4.30ppm, 4.31ppm, 4.32ppm, 4.33ppm, 4.34ppm, 4.35ppm, 4.36ppm, 4.37ppm, 4.38ppm, 4.39ppm, 4.40ppm, 4.41ppm, 4.42ppm, 4.43ppm, 4.44ppm, 4.45ppm, 4.46ppm, 4.47ppm, 4.48ppm, or any range therebetween.

In one specific embodiment described herein, in the graft compound, at least a portion of the carboxyl groups of hyaluronic acid or a salt thereof are linked to the amino groups of glutamic acid or a salt thereof via amide bonds, the graft compound has a grafting ratio of 20% to 95%, and has a general formula as shown in the following Formula (I): 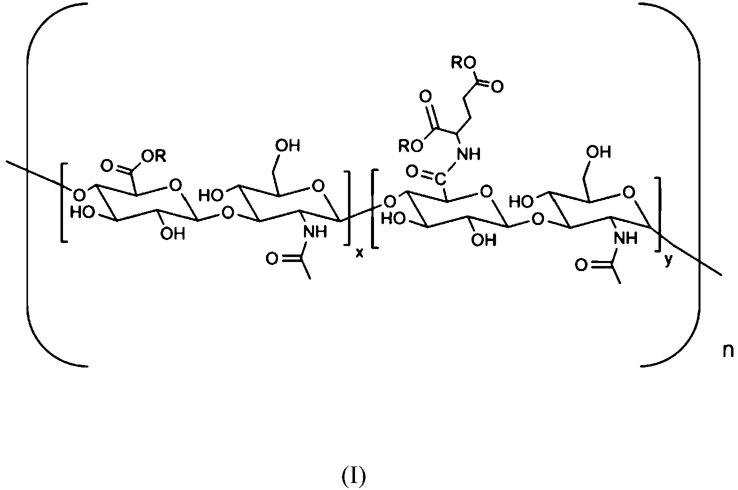 wherein in Formula (I), R is a metal ion or H, 0≤x<1, 0<y≤1, x+y=1, n is a positive integer of 1 to 2000.

In some embodiments described herein, the numerical values of x, y, grafting ratio y/x+y, and n can be described reference to the above contents.

In one specific embodiment described herein, in the graft compound, at least a portion of the carboxyl groups of hyaluronic acid or a salt thereof are linked to the amino groups of glutamic acid or a salt thereof via amide bonds, the graft compound has a grafting ratio of 20% to 100%, and has a general formula as shown in Formula (II): 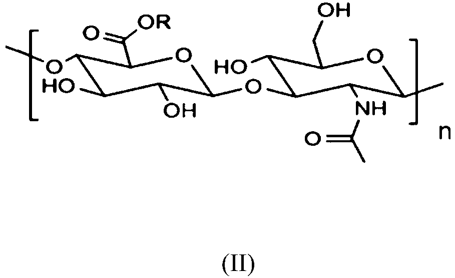 wherein in Formula (II), R is a metal ion or H, n is a positive integer of 1 to 2000, at least a portion of ORs are substituted by glutamic acid, and the substituent of glutamic acid is shown as Formula (III): 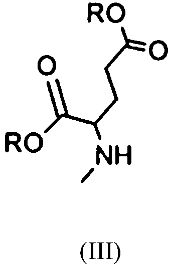 wherein in Formula (III), R is a metal ion or H.

In one specific embodiment described herein, in the graft compound, at least a portion of the carboxyl groups of hyaluronic acid or a salt thereof are linked to the amino groups of glutamic acid or a salt thereof via amide bonds, the graft compound has a grafting ratio of 20% to 100% and a molecular weight of 3kDa to 500kDa; in the ¹H NMR spectrum obtained by nuclear magnetic resonance detection of the graft compound, the graft compound has a characteristic peak at chemical shift δ of 4.00-4.15 ppm.

In one specific embodiment described herein, in the graft compound, at least a portion of the carboxyl groups of hyaluronic acid or a salt thereof are linked to the amino groups of glutamic acid or a salt thereof via amide bonds, the graft compound has a grafting ratio of 20% to 100% and a molecular weight of 3kDa to 500kDa; in the ¹H NMR spectrum obtained by nuclear magnetic resonance detection of the graft compound, the graft compound has characteristic peaks at chemical shift δ of 4.00-4.15 ppm, 2.10-2.25 ppm and 1.71-2.10 ppm.

The method for preparing the graft compound of hyaluronic acid or a salt thereof and glutamic acid or a salt thereof described herein is not specifically limited, the graft compound can be prepared according to conventional method in the art, for example by: mixing hyaluronic acid or a salt thereof with a quaternary ammonium base to react to obtain the quaternary ammonium salt of hyaluronic acid or a salt thereof; dissolving the quaternary ammonium salt of hyaluronic acid or a salt thereof, then adding glutamic acid or a salt thereof, activator and catalyst for reaction, hydrolyzing the resulting mixture under alkaline condition, and then adjusting pH, thereby obtaining the graft compound (HA-GLU) of hyaluronic acid or a salt thereof and glutamic acid or a salt thereof.

In one specific embodiment described herein, the quaternary ammonium base includes tetrabutylammonium hydroxide.

In one specific embodiment described herein, the glutamic acid or a salt thereof includes diethyl L-glutamate hydrochloride.

In one specific embodiment described herein, the activator includes 2-chloro-1-methylpyridine iodide. In one specific embodiment described herein, the catalyst includes triethylamine.

In one specific embodiment described herein, the quaternary ammonium salt of hyaluronic acid or a salt thereof is dissolved with organic solvents, and the organic solvent includes N,N-dimethylformamide (DMF).

In some embodiments described herein, the mass ratio of the quaternary ammonium salt of hyaluronic acid or a salt thereof to glutamic acid or a salt thereof is 1: (0.01-1); e.g., the mass ratio can be 1:0.01, 1:0.01, 1:0.02, 1:0.03, 1:0.04, 1:0.05, 1:0.06, 1:0.07, 1:0.08, 1:0.09, 1:0.1, 1:0.2, 1:0.3, 1:0.4, 1:0.5, 1:0.6, 1:0.7, 1:0.8, 1:0.9, 1:1, or any range therebetween.

In some embodiments described herein, the mass ratio of glutamic acid or a salt thereof, activator and catalyst is 1: (0.1-10): (0.1-5); e.g., the mass ratio can be 1:0.1:0.1, 1:0.1:0.2, 1:0.1:0.3, 1:0.1:0.4, 1:0.1:0.5, 1:0.1:0.6, 1:0.1:0.7, 1:0.1:0.8, 1:0.1:0.9, 1:0.1:1.0, 1:0.1:2.0, 1:0.1:3.0, 1:0.1:4.0, 1:0.1:5.0, 1:0.2:0.1, 1:0.2:0.2, 1:0.2:0.3, 1:0.2:0.4, 1:0.2:0.5, 1:0.2:0.6, 1:0.2:0.7, 1:0.2:0.8, 1:0.2:0.9, 1:0.2:1.0, 1:0.2:2.0, 1:0.2:3.0, 1:0.2:4.0, 1:0.2:5.0, 1:0.5:0.1, 1:0.5:0.2, 1:0.5:0.3, 1:0.5:0.4, 1:0.5:0.5, 1:0.5:0.6, 1:0.5:0.7, 1:0.5:0.8, 1:0.5:0.9, 1:0.5:1.0, 1:0.5:2.0, 1:0.5:3.0, 1:0.5:4.0, 1:0.5:5.0, 1:1.0:0.1, 1:1.0:0.2, 1:1.0:0.3, 1:1.0:0.4, 1:1.0:0.5, 1:1.0:0.6, 1:1.0:0.7, 1:1.0:0.8, 1:1.0:0.9, 1:1.0:1.0, 1:1.0:2.0, 1:1.0:3.0, 1:1.0:4.0, 1:1.0:5.0, 1:2.0:0.1, 1:2.0:0.2, 1:2.0:0.3, 1:2.0:0.4, 1:2.0:0.5, 1:2.0:0.6, 1:2.0:0.7, 1:2.0:0.8, 1:2.0:0.9, 1:2.0:1.0, 1:2.0:2.0, 1:2.0:3.0, 1:2.0:4.0, 1:2.0:5.0, 1:5.0:0.1, 1:5.0:0.2, 1:5.0:0.3, 1:5.0:0.4, 1:5.0:0.5, 1:5.0:0.6, 1:5.0:0.7, 1:5.0:0.8, 1:5.0:0.9, 1:5.0:1.0, 1:5.0:2.0, 1:5.0:3.0, 1:5.0:4.0, 1:5.0:5.0, 1:10:0.1, 1:10:0.2, 1:10:0.3, 1:10:0.4, 1:10:0.5, 1:10:0.6, 1:10:0.7, 1:10:0.8, 1:10:0.9, 1:10:1.0, 1:10:2.0, 1:10:3.0, 1:10:4.0, 1:10:5.0, or any range therebetween.

In one specific embodiment described herein, the hyaluronic acid or a salt thereof is mixed with tetrabutylammonium hydroxide, after reaction, TBA salt of the hyaluronic acid or a salt thereof (HA-TBA) is obtained; the TBA salt of the hyaluronic acid or a salt thereof is dissolved in N,N-dimethylformamide (DMF), and then 2-chloro-1-methylpyridine iodide, diethyl L-glutamate hydrochloride, and triethylamine are added for reaction, the resulting mixture is hydrolyzed under alkaline condition, the pH is adjusted, and the graft compound (HA-GLU) of hyaluronic acid or a salt thereof and glutamic acid or a salt thereof is obtained.

In one specific embodiment described herein, the hyaluronic acid or a salt thereof is mixed with tetrabutylammonium hydroxide, after reaction, TBA salt of the hyaluronic acid or a salt thereof (HA-TBA) is obtained; the TBA salt of the hyaluronic acid or a salt thereof is dissolved in N,N-dimethylformamide (DMF), and then 2-chloro-1-methylpyridine iodide, diethyl L-glutamate hydrochloride, and triethylamine are added for reaction, the resulting mixture is hydrolyzed under alkaline condition, the pH is adjusted, and the graft compound (HA-GLU) of hyaluronic acid or a salt thereof and glutamic acid or a salt thereof is obtained. The mass ratio of the quaternary ammonium salt of the hyaluronic acid or a salt thereof to the glutamic acid or a salt thereof is 1: (0.01-1), the mass ratio of diethyl L-glutamate hydrochloride, 2-chloro-1-methylpyridine iodide and triethylamine is 1: (0.1-10): (0.1-5).

Also provided herein is a cosmetic composition, the cosmetic composition can, e.g., be one of a composition for preventing and/or alleviating skin discomfort, a composition for preventing and/or alleviating skin discomfort caused by inflammation, anti-inflammatory composition and the like. The composition includes the graft compound described herein.

Provided herein is use of the above graft compound or the above composition in cosmetics.

Provided herein is use of the above graft compound or the above composition in preventing and/or alleviating skin discomfort caused by inflammation.

Provided herein is use of the above graft compound or the above composition in anti-inflammation.

Provided herein is use of the above graft compound or the above composition in preparing a product for preventing and/or alleviating skin discomfort caused by inflammation.

Provided herein is use of the above graft compound or the above composition in preparing an anti-inflammatory product.

Provided herein is use of the above graft compound or the above composition in preventing and/or alleviating skin discomfort caused by inflammation for non-therapeutic purpose.

Provided herein is use of the above graft compound or the above composition in anti-inflammation for non-therapeutic purpose.

Provided herein is use of the above graft compound or the above composition in inhibiting the expression of IL-6.

Provided herein is use of the above graft compound or the above composition in preparing a product for inhibiting the expression of IL-6.

Provided herein is use of the above graft compound or the above composition in inhibiting the expression of IL-6 for non-therapeutic purpose.

In one specific embodiment described herein, the skin discomfort caused by inflammation (e.g., IL-6) includes dermatitis, eczema, acne, vitiligo, sebum secretion, scars, skin aging, etc.

Provided herein is a method for preventing and/or alleviating skin discomfort caused by inflammation, comprising administrating the graft compound described herein or the composition described herein to a subject in need thereof.

Provided herein is a method of inhibiting the expression of IL-6, comprising administrating the graft compound described herein or the composition described herein to a subject in need thereof.

Provided herein is an anti-inflammatory method, comprising administrating the graft compound described herein or the composition described herein to a subject in need thereof.

As used herein, the term "subject" usually refers to cells or animals, which may be mammals such as humans, non-human primates (apes, gibbons, gorillas, chimpanzees, orangutans, macaques), domestic animals (dogs and cats), farm animals (poultry such as chickens and ducks, horses, cattle, goats, sheep, pigs), and laboratory animals (mice, rats, rabbits, guinea pigs). Human subjects include fetus, newborn, infant, adolescent, and adult subjects. Subjects also include animal disease models and other animal models known to those skilled in the art.

### Example

### Source of Experimental Raw Materials

**Table 1**

| Raw Material | Specific Information |
|---|---|
| UMW-HA | 3k Da, purity>99%, batch number: 21011446, Bloomage Biotechnology Corporation Limited |
| LMW-HA | 4.7w Da, purity>99%, batch number: 21011446, Bloomage Biotechnology Corporation Limited |
| MMW-HA | 36.8w Da, purity>99%, batch number: 20110642, Bloomage Biotechnology Corporation Limited |
| HMW-HA | 85w Da, purity>99%, batch number: 22021741, Bloomage Biotechnology Corporation Limited |
| GLU | glutamic acid, batch number: 20210224, Sinopharm Chemical Reagent Co., Ltd |
| diethyl L-glutamate hydrochloride | batch number: C14511820, Shanghai Macklin Biochemical Co., Ltd |
| tetrabutylammonium hydroxide | batch number: F2110138, Shanghai Aladdin Biochemical Technology Co., Ltd |
| triethylamine | batch number: 20220121, Sinopharm Chemical Reagent Co., Ltd |
| 2-chloro-1-methylpyridine iodide | batch number: C15143497, Shanghai Macklin Biochemical Co., Ltd |
| N,N-dimethylformamide | batch number: 20220718, Tianjin Fuyu Fine Chemical Co., Ltd |
| fibroblast | batch number: Fb22113007, Guangdong BioCell Biotechnology Co.Ltd |
| DMEM basic medium | Gibco, Thermo Fisher Scientific |
| D'Hanks buffer | Gibco, Thermo Fisher Scientific |
| PS bispecific antibody solution | Gibco, Thermo Fisher Scientific |
| FBS fetal bovine serum | Gibco, Thermo Fisher Scientific |
| reverse transcription kit | RNA-Quick Purification Kit, Yishan Biotech |

### Example 1 Preparation of Graft-1 sample

Low molecular weight hyaluronic acid (designated as LMW-HA) was swollen in ethanol, acidified, washed with anhydrous ethanol, and vacuum filtered. The filter cake was dissolved in water, and the pH was adjusted to neutral by using tetrabutylammonium hydroxide. After vacuum filtration and drying, the solid TBA salt of hyaluronic acid (designated as HA-TBA) was obtained. The molecular weight of the low molecular weight hyaluronic acid was 4.7 wDa.

2 g of HA-TBA was dissolved into N,N-dimethylformamide (DMF). After completing the dissolution, 0.79 g of 2-chloro-1-methylpyridine iodide (CMPI), 0.15 g of diethyl L-glutamate hydrochloride, and 0.32 g of triethylamine were added into the system, and were stirred at room temperature overnight. After completing the reaction, purified water and base were added to the reaction system for hydrolysis. Then the pH was adjusted to weak acidic with acid, followed by precipitated and purified by using organic solvent. The filtrate was vacuum filtered to obtain a filter cake, and dried to obtain the sample, which was designated as Graft-1.

### Example 2 Preparation of Graft-2 sample

Low molecular weight hyaluronic acid (designated as LMW-HA) was swollen in ethanol, acidified, washed with anhydrous ethanol, and vacuum filtered. The filter cake was dissolved in water, and the pH was adjusted to neutral by using tetrabutylammonium hydroxide. After vacuum filtration and drying, the solid TBA salt of hyaluronic acid (designated as HA-TBA) was obtained. The molecular weight of the low molecular weight hyaluronic acid was 4.7 wDa.

2 g of HA-TBA was dissolved into N,N-dimethylformamide (DMF). After completing the dissolution, 0.79 g of 2-chloro-1-methylpyridine iodide (CMPI), 0.23 g of diethyl L-glutamate hydrochloride, and 0.48 g of triethylamine were added into the system, and were stirred at room temperature overnight. After completing the reaction, purified water and base were added to the reaction system for hydrolysis. Then the pH was adjusted to weak acidic with acid, followed by precipitated and purified by using organic solvent. The filtrate was vacuum filtered to obtain a filter cake, and dried to obtain the sample, which was designated as Graft-2.

### Example 3 Preparation of Graft-3 sample

Low molecular weight hyaluronic acid (designated as LMW-HA) was swollen in ethanol, acidified, washed with anhydrous ethanol, and vacuum filtered. The filter cake was dissolved in water, and the pH was adjusted to neutral by using tetrabutylammonium hydroxide. After vacuum filtration and drying, the solid TBA salt of hyaluronic acid (designated as HA-TBA) was obtained. The molecular weight of the low molecular weight hyaluronic acid was 4.7 wDa.

2 g of HA-TBA was dissolved into N,N-dimethylformamide (DMF). After completing the dissolution, 0.79 g of 2-chloro-1-methylpyridine iodide (CMPI), 0.38 g of diethyl L-glutamate hydrochloride, and 0.56 g of triethylamine were added into the system, and were stirred at room temperature overnight. After completing the reaction, purified water and base were added to the reaction system for hydrolysis. Then the pH was adjusted to weak acidic with acid, followed by precipitated and purified by using organic solvent. The filtrate was vacuum filtered to obtain a filter cake, and dried to obtain the sample, which was designated as Graft-3.

### Example 4 Preparation of Graft-4 sample

Low molecular weight hyaluronic acid (designated as LMW-HA) was swollen in ethanol, acidified, washed with anhydrous ethanol, and vacuum filtered. The filter cake was dissolved in water, and the pH was adjusted to neutral by using tetrabutylammonium hydroxide. After vacuum filtration and drying, the solid TBA salt of hyaluronic acid (designated as HA-TBA) was obtained. The molecular weight of the low molecular weight hyaluronic acid was 4.7 wDa.

2 g of HA-TBA was dissolved into N,N-dimethylformamide (DMF). After completing the dissolution, 0.79 g of 2-chloro-1-methylpyridine iodide (CMPI), 0.46 g of diethyl L-glutamate hydrochloride, and 0.56 g of triethylamine were added into the system, and were stirred at room temperature overnight. After completing the reaction, purified water and base were added to the reaction system for hydrolysis. Then the pH was adjusted to weak acidic with acid, followed by precipitated and purified by using organic solvent. The filtrate was vacuum filtered to obtain a filter cake, and dried to obtain the sample, which was designated as Graft-4.

### Example 5 Preparation of Graft-5 sample

Low molecular weight hyaluronic acid (designated as LMW-HA) was swollen in ethanol, acidified, washed with anhydrous ethanol, and vacuum filtered. The filter cake was dissolved in water, and the pH was adjusted to neutral by using tetrabutylammonium hydroxide. After vacuum filtration and drying, the solid TBA salt of hyaluronic acid (designated as HA-TBA) was obtained. The molecular weight of the low molecular weight hyaluronic acid was 4.7 wDa.

2 g of HA-TBA was dissolved into N,N-dimethylformamide (DMF). After completing the dissolution, 0.79 g of 2-chloro-1-methylpyridine iodide (CMPI), 0.54 g of diethyl L-glutamate hydrochloride, and 0.62 g of triethylamine were added into the system, and were stirred at room temperature overnight. After completing the reaction, purified water and base were added to the reaction system for hydrolysis. Then the pH was adjusted to weak acidic with acid, followed by precipitated and purified by using organic solvent. The filtrate was vacuum filtered to obtain a filter cake, and dried to obtain the sample, which was designated as Graft-5.

### Example 6 Preparation of Graft-6 sample

Low molecular weight hyaluronic acid (designated as LMW-HA) was swollen in ethanol, acidified, washed with anhydrous ethanol, and vacuum filtered. The filter cake was dissolved in water, and the pH was adjusted to neutral by using tetrabutylammonium hydroxide. After vacuum filtration and drying, the solid TBA salt of hyaluronic acid (designated as HA-TBA) was obtained. The molecular weight of the low molecular weight hyaluronic acid was 4.7 wDa.

2 g of HA-TBA was dissolved into N,N-dimethylformamide (DMF). After completing the dissolution, 0.79 g of 2-chloro-1-methylpyridine iodide (CMPI), 1 g of diethyl L-glutamate hydrochloride, and 0.81 g of triethylamine were added into the system, and were stirred at room temperature overnight. After completing the reaction, purified water and base were added to the reaction system for hydrolysis. Then the pH was adjusted to weak acidic with acid, followed by precipitated and purified by using organic solvent. The filtrate was vacuum filtered to obtain a filter cake, and dried to obtain the sample, which was designated as Graft-6.

### Example 7 Preparation of Graft-7 sample

Small molecular weight hyaluronic acid (designated as UMW-HA) was swollen in ethanol, acidified, washed with anhydrous ethanol, and vacuum filtered. The filter cake was dissolved in water, and the pH was adjusted to neutral by using tetrabutylammonium hydroxide. After vacuum filtration and drying, the solid TBA salt of hyaluronic acid (designated as HA-TBA) was obtained. The molecular weight of the small molecular weight hyaluronic acid was 3kDa.

2 g of HA-TBA was dissolved into N,N-dimethylformamide (DMF). After completing the dissolution, 0.79 g of 2-chloro-1-methylpyridine iodide (CMPI), 0.46 g of diethyl L-glutamate hydrochloride, and 0.56 g of triethylamine were added into the system, and were stirred at room temperature overnight. After completing the reaction, purified water and base were added to the reaction system for hydrolysis. Then the pH was adjusted to weak acidic with acid, followed by precipitated and purified by using organic solvent. The filtrate was vacuum filtered to obtain a filter cake, and dried to obtain the sample, which was designated as Graft-7.

### Example 8 Preparation of Graft-8 sample

Medium molecular weight hyaluronic acid (designated as MMW-HA) was swollen in ethanol, acidified, washed with anhydrous ethanol, and vacuum filtered. The filter cake was dissolved in water, and the pH was adjusted to neutral by using tetrabutylammonium hydroxide. After vacuum filtration and drying, the solid TBA salt of hyaluronic acid (designated as HA-TBA) was obtained. The molecular weight of the medium molecular weight hyaluronic acid was 36.8wDa.

2 g of HA-TBA was dissolved into N,N-dimethylformamide (DMF). After completing the dissolution, 0.79 g of 2-chloro-1-methylpyridine iodide (CMPI), 0.46 g of diethyl L-glutamate hydrochloride, and 0.56 g of triethylamine were added into the system, and were stirred at room temperature overnight. After completing the reaction, purified water and base were added to the reaction system for hydrolysis. Then the pH was adjusted to weak acidic with acid, followed by precipitated and purified by using organic solvent. The filtrate was vacuum filtered to obtain a filter cake, and dried to obtain the sample, which was designated as Graft-8.

### Reference Example 9 Preparation of Graft-9 sample

High molecular weight hyaluronic acid (designated as HMW-HA) was swollen in ethanol, acidified, washed with anhydrous ethanol, and vacuum filtered. The filter cake was dissolved in water, and the pH was adjusted to neutral by using tetrabutylammonium hydroxide. After vacuum filtration and drying, the solid TBA salt of hyaluronic acid (designated as HA-TBA) was obtained. The molecular weight of the high molecular weight hyaluronic acid was 85wDa.

2 g of HA-TBA was dissolved into N,N-dimethylformamide (DMF). After completing the dissolution, 0.79 g of 2-chloro-1-methylpyridine iodide (CMPI), 0.46 g of diethyl L-glutamate hydrochloride, and 0.56 g of triethylamine were added into the system, and were stirred at room temperature overnight. After completing the reaction, purified water and base were added to the reaction system for hydrolysis. Then the pH was adjusted to weak acidic with acid, followed by precipitated and purified by using organic solvent. The filtrate was vacuum filtered to obtain a filter cake, and dried to obtain the sample, which was designated as Graft-9.

**Table 2 Parameters of Examples and Reference Examples**

| Example | designation | Molecular weight of HA Da | Grafting ratio | Total molecular weight of HA-GLU Da |
|---|---|---|---|---|
| Example 1 | Graft-1 | 4.7w | 12% | 4.71w |
| Example 2 | Graft-2 | 4.7w | 27% | 4.73w |
| Example 3 | Graft-3 | 4.7w | 34% | 4.73w |
| Example 4 | Graft-4 | 4.7w | 47% | 4.75w |
| Example 5 | Graft-5 | 4.7w | 65% | 4.77w |
| Example 6 | Graft-6 | 4.7w | 87% | 4.88w |
| Example 7 | Graft-7 | 3k | 47% | 3.1k |
| Example 8 | Graft-8 | 36.8w | 47% | 38.1w |
| Reference Example 9 | Graft-9 | 85w | 47% | 87.7w |

| | | | | |
|---|---|---|---|---|
| Note: HA-GLU refers to the graft compound of hyaluronic acid-glutamic acid. | | | | |

### Experimental Example

### Experimental Example 1

10 mg of samples from Examples 1-8 and Reference Example 9, along with 10 mg of hyaluronic acid sample having molecular weight of 4.7 wDa and 10 mg of glutamic acid sample were weighed and dissolved in deuterium oxide (D₂O) to form a clear and transparent solution. An appropriate amount of the transparent solution was taken and transferred into an NMR tube. The hydrogen spectrum was scanned using a nuclear magnetic resonance spectrometer, wherein the number of scans was set to be 16, the solvent was selected to be D₂O, and the button "Start Test" was clicked. ¹H NMR hydrogen spectra of HA-GLUs with different grafting ratios were obtained.

Wherein, the ¹H NMR spectrum of Example 6 is shown in FIG. 1.

Wherein, the specific analysis results of the characteristic peaks shown by Examples 1-8, Reference Example 9, hyaluronic acid and glutamic acid are shown in Table 3.

**Table 3**

| Materials | Chemical Shift (ppm) | Assignment (monomer/graft compound marker) | Coupling splitting | Relative proton ratio |
|---|---|---|---|---|
| HA | 1.936 | 6 | 1 | 3 |
| | 4.376 | 1 | 1 | 1 |
| | 4.474 | 1' | 1 | 1 |
| GLU | 1.778-1.976 | 2 | m | 2 |
| | 2.153 | 3 | 3 | 2 |
| | 3.537 | 1 | 1 | 1 |
| Graft-1 | 1.767-2.055 | 7, 9 | m | 3.27 |
| | 2.189 | 8 | 3 | 0.25 |
| | 4.102 | 6 | 1 | 0.12 |
| | 4.371 | 1 | 1 | 1 |
| | 4.459 | 1' | 1 | 1 |
| Graft-2 | 1.777-2.085 | 7, 9 | m | 3.52 |
| | 2.193 | 8 | 3 | 0.53 |
| | 4.104 | 6 | 1 | 0.27 |
| | 4.371 | 1 | 1 | 1 |
| | 4.463 | 1' | 1 | 1 |
| Graft-3 | 1.716-2.085 | 7, 9 | m | 3.66 |
| | 2.192 | 8 | 3 | 0.66 |
| | 4.101 | 6 | 1 | 0.34 |
| | 4.371 | 1 | 1 | 1 |
| | 4.463 | 1' | 1 | 1 |
| Graft-4 | 1.833-2.069 | 7, 9 | m | 3.98 |
| | 2.185 | 8 | 3 | 0.97 |
| | 4.096 | 6 | 1 | 0.47 |
| | 4.376 | 1 | 1 | 1 |
| | 4.463 | 1' | 1 | 1 |
| Graft-5 | 1.833-2.069 | 7, 9 | m | 4.28 |
| | 2.221 | 8 | 3 | 1.25 |
| | 4.115 | 6 | 1 | 0.65 |
| | 4.376 | 1 | 1 | 1 |
| | 4.464 | 1' | 1 | 1 |
| Graft-6 | 1.786-1.995 | 7, 9 | m | 4.74 |
| | 2.215 | 8 | 3 | 1.73 |
| | 4.082 | 6 | 1 | 0.87 |
| | 4.318 | 1 | 1 | 1 |
| | 4.409 | 1' | 1 | 1 |
| Graft-7 | 1.815-2.063 | 7, 9 | m | 3.96 |
| | 2.183 | 8 | 3 | 0.95 |
| | 4.085 | 6 | 1 | 0.47 |
| | 4.326 | 1 | 1 | 1 |
| | 4.453 | 1' | 1 | 1 |
| Graft-8 | 1.845-2.062 | 7, 9 | m | 3.96 |
| | 2.183 | 8 | 3 | 0.95 |
| | 4.084 | 6 | 1 | 0.47 |
| | 4.312 | 1 | 1 | 1 |
| | 4.433 | 1' | 1 | 1 |
| Graft-9 | 1.825-2.068 | 7, 9 | m | 3.99 |
| | 2.189 | 8 | 3 | 0.97 |
| | 4.084 | 6 | 1 | 0.47 |
| | 4.326 | 1 | 1 | 1 |
| | 4.453 | 1' | 1 | 1 |

| | | | | |
|---|---|---|---|---|
| Chemical Shift: refers to chemical shift; Coupling splitting refers to coupling splitting multiplet number; Relative proton ratio refers to relative proton number. | | | | |

It can be seen from Table 3 and the ¹H NMR spectrum obtained by nuclear magnetic resonance detection of the graft compound shown in FIG. 1, compared to glutamic acid, the chemical shifts of the methine groups and methylene groups derived from glutamic acid in the graft compound both shift toward the low-field region.

The characteristic peaks of the methylene groups from glutamic acid or a salt thereof appear at chemical shifts δ = 1.71-2.10 ppm and δ = 2.10-2.25 ppm, respectively; preferably, the characteristic peaks of the methine groups from glutamic acid or a salt thereof appear at chemical shifts δ=4.00-4.15ppm; the characteristic peaks of -CH- from hyaluronic acid or a salt thereof appear at chemical shifts δ=4.30-4.40ppm and δ=4.40-4.48ppm, respectively.

The calculation of the grafting ratio of the HA-GLU graft compound:
The grafting molar ratio of the graft compound was determined by measuring the integral area in the hydrogen spectrum of glutamic acid via nuclear magnetic resonance spectroscopy ¹H NMR, thereby determining the grafting ratio of the HA-GLU molecule.

Specifically, the grafting ratio was determined by measuring the peak area integral ∫ of the methine group -CH- proton (shifts toward 4.05-4.12 ppm) brought by glutamic acid in the graft compound (if the grafting ratio is 100%, the integral area at this position is 1).

**Table 4**

| No. | Grafting ratio% | ∫ (f1 = 4.05 -4.12) | ∫ (f2.15-2.25) | ∫ (f1.76-2.07) |
|---|---|---|---|---|
| Graft-1 | 12 | 0.12 | 0.25 | 3.27 |
| Graft-2 | 27 | 0.27 | 0.53 | 3.52 |
| Graft-3 | 34 | 0.34 | 0.66 | 3.66 |
| Graft-4 | 47 | 0.47 | 0.97 | 3.98 |
| Graft-5 | 65 | 0.65 | 1.25 | 4.28 |
| Graft-6 | 87 | 0.87 | 1.73 | 4.74 |
| Graft-7 | 47 | 0.47 | 0.95 | 3.96 |
| Graft-8 | 47 | 0.47 | 0.96 | 3.96 |
| Graft-9 | 47 | 0.47 | 0.97 | 3.99 |

### Experimental Example 2

Complete medium solution was firstly formulated: DMEM basic medium, FBS fetal bovine serum, and PS bispecific antibody solution were mixed in a mass ratio of 89: 10: 1.

0.009 g of UMW-HA, LMW-HA, MMW-HA, HMW-HA, GLU, physical mixture, and Graft-1~Graft-9 powder were weighed, respectively, and then were dissolved in the complete medium solution, to form a mixture to be tested, the concentrations of which were all 150 µg/mL.

Wherein, No. 1 refers to the medium solution to which UMW-HA is added, wherein the concentration of UMW-HA is 150 µg/mL;
No. 2 refers to the medium solution to which LMW-HA is added, wherein the concentration of LMW-HA is 150 µg/mL;
No. 3 refers to the medium solution to which MMW-HA is added, wherein the concentration of MMW-HA is 150 µg/mL;
No. 4 refers to the medium solution to which HMW-HA is added, wherein the concentration of HMW-HA is 150 µg/mL;
No. 5 refers to the medium solution to which GLU is added, wherein the concentration of GLU is 150 µg/mL;
No. 6 refers to the medium solution to which LMW-HA and GLU are added, wherein the concentration of LMW-HA is 136 µg/mL, and the concentration of GLU is 14 µg/mL;
No. 7 refers to the medium solution to which Graft-1 is added, wherein the concentration of Graft-1 is 150 µg/mL;
No. 8 refers to the medium solution to which Graft-2 is added, wherein the concentration of Graft-2 is 150 µg/mL;
No. 9 refers to the medium solution to which Graft-3 is added, wherein the concentration of Graft-3 is 150 µg/mL;
No. 10 refers to the medium solution to which Graft-4 is added, wherein the concentration of Graft-4 is 150 µg/mL;
No. 11 refers to the medium solution to which Graft-5 is added, wherein the concentration of Graft-5 is 150 µg/mL;
No. 12 refers to the medium solution to which Graft-6 is added, wherein the concentration of Graft-6 is 150 µg/mL;
No. 13 refers to the medium solution to which Graft-7 is added, wherein the concentration of Graft-7 is 150 µg/mL;
No. 14 refers to the medium solution to which Graft-8 is added, wherein the concentration of Graft-8 is 150 µg/mL;
No. 15 refers to the medium solution to which Graft-9 is added, wherein the concentration of Graft-9 is 150 µg/mL.

The cytology testing method is as follows:
1) Cell seeding: the fibroblasts were inoculated at a seeding density of 1 × 10⁵ cells/well into a 6-well plate, and incubated in an incubator (37°C, 5%CO₂) for 24 hrs.
2) Modeling: according to experimental group, the sample groups and model control group were exposed to UVB phototherapy device (wavelength of 280-320nm, 80mJ/cm²) for UVB irradiation.
3) Dosing: the groups was dosed according to the testing protocol recorded in Table 5, the complete medium containing working solution of the test materials was added into each well of the sample groups, with 1 ml of sample per well and three replicate wells per group; after dosing, the 6-well plate was placed in an incubator (37°C, 5% CO₂) and incubated for 24hrs.
4) Cell collecting: after incubating for 24hrs, the cell supernatants were collected, then washed twice with 1 mL/well of D'Hanks buffer, after blowing and lysing the cells, the sample was collected.
5) Gene expression testing: after treating the cells with the RNA-Quick Purification Kit, the samples were collected; following the instructions of the kit, RNA extraction, reverse transcription, and fluorescence quantitative PCR operations were performed; the results were calculated by using the 2^{-△△Ct} method, to obtain the test results.

The test results are shown in Table 5, and the standard deviation of the experimental test is 0.01. The lower the relative expression level of IL-6, the stronger the effect of the test sample on inhibiting the expression of IL-6. Compared to hyaluronic acid or glutamic acid alone, if the relative expression of IL-6 is lower in the graft compound at identical concentration, this indicates that the graft compound exhibits a synergistic effect. Compared to an equivalent amount of hyaluronic acid or glutamic acid as a single component, the graft compound described herein demonstrated reduced relative expression of IL-6; compared to an equivalent amount of a physical mixture of the two components, the graft compound described herein also demonstrated reduced relative expression of IL-6; this indicates that the graft compound exhibits a synergistic effect.

**Table 5**

| No | Formulations for cell tests | Relative expression level of IL-6 |
|---|---|---|
| NC | NA | 1.000 |
| 1 | 150 µg/mL UMW-HA | 0.518 |
| 2 | 150 µg/mL LMW-HA | 0.538 |
| 3 | 150 µg/mL MMW-HA | 0.717 |
| 4 | 150 µg/mL HMW-HA | 0.562 |
| 5 | 150 µg/mL GLU | 0.753 |
| 6 | 136 µg/mL LMW-HA +14 µg/mL GLU | 0.775 |
| 7 | 150 µg/mL Graft-1 | 0.582 |
| 8 | 150 µg/mL Graft-2 | 0.395 |
| 9 | 150 µg/mL Graft-3 | 0.357 |
| 10 | 150 µg/mL Graft-4 | 0.132 |
| 11 | 150 µg/mL Graft-5 | 0.165 |
| 12 | 150 µg/mL Graft-6 | 0.522 |
| 13 | 150 µg/mL Graft-7 | 0.152 |
| 14 | 150 µg/mL Graft-8 | 0.302 |
| 15 | 150 µg/mL Graft-9 | 0.880 |

Although the present invention has been described in detail above with reference to examples, such disclosure is not intended to limit the scope of the present invention. Those skilled in the art may make various changes and modifications without departing from the spirit and scope of the present invention. Therefore, the protection scope of the present invention shall be determined by the appended claims.

## Claims

1. A graft compound of hyaluronic acid or a salt thereof and glutamic acid or a salt thereof, wherein at least a portion of the carboxyl groups of the hyaluronic acid or a salt thereof are linked to the amino groups of the glutamic acid or a salt thereof via amide bonds.

2. The graft compound according to claim 1, having a general formula as shown in the following Formula (I): 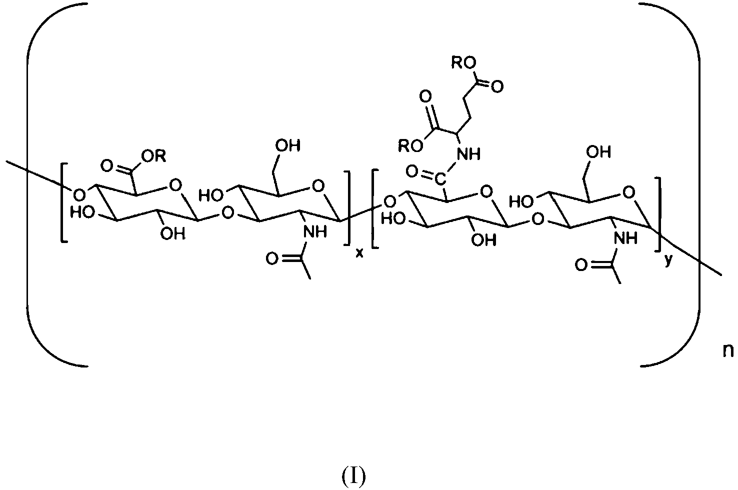 wherein in Formula (I), R is a metal ion or H, 0≤x<1, 0<y≤1, x+y=1, n is a positive integer of 1 to 2000.

3. The graft compound according to claim 1, wherein the grafting ratio of the graft compound is 20% to 100%.

4. The graft compound according to claim 1, wherein the molecular weight of the graft compound is 3kDa to 500kDa.

5. The graft compound according to claim 1, wherein in the ¹H NMR spectrum obtained by nuclear magnetic resonance detection of the graft compound, the graft compound has a characteristic peak at chemical shift δ of 4.00-4.15 ppm;
preferably, in the ¹H NMR spectrum obtained by nuclear magnetic resonance detection of the graft compound, the graft compound has characteristic peaks at chemical shift δ of 2.10-2.25 ppm and 1.71-2.10 ppm.

6. A method for preparing the graft compound of hyaluronic acid or a salt thereof and glutamic acid or a salt thereof according to any one of claims 1 to 5, wherein
the quaternary ammonium salt of hyaluronic acid or a salt thereof is prepared by: mixing hyaluronic acid or a salt thereof with a quaternary ammonium base to react to obtain the quaternary ammonium salt of hyaluronic acid or a salt thereof;
the graft compound is prepared by: dissolving the quaternary ammonium salt of hyaluronic acid or a salt thereof, then adding glutamic acid or a salt thereof, activator and catalyst for reaction, hydrolyzing the resulting mixture under alkaline condition, and then adjusting pH, thereby obtaining the graft compound of hyaluronic acid or a salt thereof and glutamic acid or a salt thereof.

7. A cosmetic composition, wherein the cosmetic composition comprises the graft compound according to any one of claims 1 to 5 or the graft compound prepared by the method of claim 6.

8. Use of the graft compound according to any one of claims 1 to 5 or the graft compound prepared by the method of claim 6 in cosmetics.

9. Use of the graft compound according to any one of claims 1 to 5 or the graft compound prepared by the method of claim 6 or the cosmetic composition according to claim 7 in preventing and/or alleviating skin discomfort caused by inflammation.

10. Use of the graft compound according to any one of claims 1 to 5 or the graft compound prepared by the method of claim 6 or the cosmetic composition according to claim 7 in anti-inflammation.
